(19) Europäisches Patentamt
European Patent Office
Office européen des brevets

(11) **EP 4 506 691 A1**

## (12) EUROPEAN PATENT APPLICATION

(43) Date of publication:
**12.02.2025 Bulletin 2025/07**

(21) Application number: **23190568.8**

(22) Date of filing: **09.08.2023**

(51) International Patent Classification (IPC):
**G01N 33/557** (2006.01)

(52) Cooperative Patent Classification (CPC):
**G01N 33/557**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Leica Microsystems CMS GmbH 35578 Wetzlar (DE)**

(72) Inventors:
• **Alsheimer, Soeren 35578 Wetzlar (DE)**
• **Bradl, Joachim 35578 Wetzlar (DE)**

(74) Representative: **Schaumburg und Partner Patentanwälte mbB Mauerkircherstraße 31 81679 München (DE)**

(54) **METHOD AND SYSTEM FOR ANALYSING A BIOLOGICAL SAMPLE**

(57) A method for analysing a biological sample comprising multiple targets is provided. The method comprises the following steps: Staining the targets of the biological sample with multiple detectable markers; Providing affinity information specifying an affinity of each marker towards at least some of the targets; Generating a readout of the biological sample with the markers to generate raw readout data; Unmixing the raw readout data by applying an unmixing algorithm and the affinity information to generate unmixed readout data with respect to the targets. In a further aspect a system for analysing a biological sample is provided.

**Fig. 3**

## Description

<u>Technical field</u>

The invention relates to a method for analysing a biological sample comprising multiple targets. In a further aspect, a system is provided comprising means configured to carry out the method.

<u>Background</u>

The determination of proteomic, genomic and transcriptomic data from biological samples remains a focus in the life sciences. Generally optical readouts are used to identify target structures that are marked with fluorescent markers and that specifically attach to target structures by means of affinity reagents. This enables the identification of proteins, for example. In combination with imaging the biological sample, the light emitted by the markers at a particularly location in the biological samples may be used to assign the identified proteins. Years of scientific effort have generated enormous body of data generated in this way.

The basic assumption underlying these efforts is that affinity reagents can be used to these ends, as they bind their exclusive targets with sufficient affinity and specificity. Accumulating evidence shows, however, that this basic tenet does not withstand scrutiny and that most affinity reagents are cross-reactive, i.e. they do not bind to their targets exclusively.

As fields like cytometry, plasma proteomics, and microscopy are evolving towards higher levels of plexity, the problem of cross-reactivity (CR) has come more into focus and has been recognized by the scientific community as a key component of the *reproducibility crisis.* In a position paper called "Reproducibility: Standardize antibodies used in research." published by Bradbury and Pluckthin in Nature 518, 27-29 (2015) with 110 cosignatories it was stated that *"...fewer than half of around 6,000 routinely used commercial antibodies recognized only their specified targets."* More recent work by Schwenk et al. has analysed 11,000 affinity-purified, monoclonal antibodies and found only 531 to produce a single band on a western blot.

The advent of more multi-plex analysis technologies such as bead-based assays or protein microarrays brought the increasing realisation that the cross-reactivity (CR) of affinity reagents, i.e. their binding to non-targets or so called OFF-targets is a fundamental limitation of this approach, with significant impact on life science research and society.

<u>Summary</u>

It is an object to provide a method and a system for analysing a biological sample with markers that have cross-reactivity to several targets.

The aforementioned object is achieved by the subject-matter of the independent claims. Advantageous embodiments are defined in the dependent claims and the following description.

A method for analysing a biological sample comprising multiple targets is provided. The method comprises the following steps: Staining or contacting or connecting the targets of the biological sample with multiple detectable markers; Providing affinity information specifying an affinity of each marker towards at least some of the targets; Generating a readout of the biological sample with the markers to generate raw readout data; Unmixing the raw readout data by applying an unmixing algorithm and the affinity information to generate unmixed readout data with respect to the targets.

Each of the multiple detectable markers comprises an affinity reagent and a detectable label bound to the affinity reagent for detecting the marker. The binding of a detectable label to the assigned affinity reagent may be performed before staining or contacting or connecting the targets of the biological sample (preconstituted marker; e.g. direct antibody-label conjugate). Alternatively, binding of the detectable label may be simultaneous to or following to introducing the affinity reagent to the sample (e.g. barcoded antibody that is subsequently bound by a barcoded label).

The affinity reagent may comprise an amino acid structure or a nucleic acid structure, for example. The amino acid structure may be an antibody or an antibody fragment, for example. Further, the amino acid structure may be a peptide-based aptamer. The nucleic acid structure may be a nucleic acid aptamer, for example. Generally, an affinity reagent, specifically an aptamer affinity reagent, has a complex secondary, tertiary and/or quaternary structure. This complex secondary, tertiary and/or quaternary structure generally mediates binding of any one affinity reagent to any one particular target. Specifically, this binding based on the complex secondary, tertiary and/or quaternary structure does not include binding based on complementary sequence hybridisation, which is dictated merely by the linear order of nucleotides in a nucleic acid sequence.

Generally, when aiming to identify a target in a biological sample with detectable markers, the affinity reagent of one of the multiple detectable markers is configured to specifically bind to one of the multiple targets. Thus, the affinity of that affinity reagent to the one target is very high compared to the remaining targets, resulting in the preferred binding of the marker to the one target. This enables identifying the target by detecting the detectable marker. By providing at least a number of specific and distinguishable markers equal to the number of targets, all targets may be identified. The addition of markers to the biological sample such that the markers bind to their respective targets is usually called staining the biological sample. For example, the affinity of an antibody is generally regarded as the strength of the binding interaction between an antigen and the antibody. However, affinity reagents frequently have affinities of measurable and considerable strengths to more than one target and therefore may also bind to more than one target. This is often termed cross-reactivity of affinity reagents. In case these more than one targets are all present in the biological sample, the affinity reagents, and their associated markers, may bind to several targets, with the target the affinity reagent is configured to specifically bind to, being only the predominant binding partner, for example. The targets that are not the predominant binding partner or target, may also be called off-target binding partners and the corresponding binding may be called off-target binding. The binding to off-target binding partners may be in the range of 1% to 20% relative to the predominant target binding, for example. In some cases, the binding to off-target binding partners may be in the range of 1% to 5%. In some other cases, the binding to off-target binding partners may be comparable to the predominant binding partner. This off-target binding usually reduces the ability to distinguish between the targets, in particular between the targets and off-targets.

The target of one of the multiple detectable markers may be a particular part or an area of a molecule that the marker binds to, for example, an epitope of a particular protein such as an antigen. In particular, each target of the multiple targets differs, at least partially, from the remaining targets of the multiple targets. The affinity information may be provided as a matrix comprising the affinity or binding behaviour of each detectable marker to each of the targets or to at least some of the targets which are the predominant targets of the other affinity reagents being used to analyse the biological sample, for example. As a measure of the affinities, the matrix may comprise dissociation constant ($K_D$) values, for example. The biological sample may be a tissue section, for example, comprising a large number of proteins-of-interest as targets.

Thus, the raw readout data generated from the readout of the biological sample with the markers may be unmixed with the unmixing algorithm and the affinity information. This enables distinguishing between the targets, despite off-target binding of the markers, in particular their respective affinity reagents.

Preferably, the targets are on at least one target molecule. For example, each target may correspond to one target molecule. In this case, each target molecule has one target, the target being a part of the target molecule that the marker, in particular the affinity reagent, binds to. This enables analysing biological sample with a large number of targets. Alternatively, a target molecule may comprise several targets. In this case, several markers, in particular the affinity reagents, bind to respective parts of the target molecules. For example, a protein may comprise several epitopes as targets, and to each epitope a respective marker may bind. This enables analysing the biological sample with an increased accuracy.

Preferably, the detectable markers for staining the targets are selected from a pool of detectable markers based on affinity information specifying the affinity of each of the detectable markers in the pool of detectable markers at least towards the targets of the biological sample. This enables selecting markers that are suitable for staining the targets. In particular, this selection is carried out prior to the step of staining the targets of the biological sample. The selection may further comprise considering the type of biological sample to be analysed and in particular the proteins in the biological sample. The affinity information may be determined with a language model, in particular the respective affinity reagents or their (amino acid) sequences that have the desired cross-reactivity characteristics, may be determined. An example of a (large) language model is Alpha-Fold.

It is particularly preferred, that the detectable markers for staining the targets are selected, in particular from the pool of detectable markers, such that the affinity of each detectable marker to more than one of the targets is below a predetermined threshold. A preferred predetermined threshold for a cross reactivity may have a value e.g. of 0.2 or 0.1, 0.05 or less. This enables selecting markers with a reduced cross-reactivity. In particular, markers may be chosen that have (high) affinity to only one target in the biological sample or to as few targets as possible given the pool of detectable markers.

The method may also take advantage of affinity reagents known to strongly bind to a set of targets, i.e. target and off-targets. For example, an affinity reagent that strongly binds to its predominant binding partner and two off-target binding partners may be used in an assay that involves further affinity reagents. To which degree a certain affinity reagent with a certain cross-reactivity profile contributes to an assay using the method depends on the cross-reactivity profiles of other affinity reagents in said assay. In a particularly preferred embodiment, a user-defined set of targets is analysed using the smallest possible set of affinity reagents in an assay that is both necessary and sufficient to yield a well-posed set of linear equations. For the sake of simplicity this noise originating from unspecific background binding, dye labelling, and readout is ignored as this can be dealt with experimentally well enough through various means, e.g. blocking, washing, using bright labels, longer acquisition times. This means that the method renders affinity reagents useful that bind to multiple targets. For example, an antibody that binds to 5 targets strongly and with comparable affinity may not be very useful in a standard immunofluorescence experiment involving just one marker comprising said antibody, as there will be no way to differentiate the signal into 5 target-specific channels. Likewise, even if more markers are used but cross-reactivity information is not used to unmix using the strategy as it is proposed in this document, then this antibody may not be very useful as the results generated in this way will be a convolution of signals. If, however, the method described in this document is used this antibody may be very useful as part of a set of markers that is used to analyse a set of targets. In other words, what matters more than the individual cross reactivity of an affinity reagent is the nature and structure of the cross-reactivity matrix of affinity information, that describes the cross reactivity of all affinity reagents that are used in an assay using the method. In this sense the modulation is the more important aspect or in other words sharp cross reactivity profiles are preferred over profiles with a lower degree of modulation. In other words, an affinity reagent that binds with a comparable affinity to a larger number of targets may not be very useful irrespective of whether this is high, medium, or low affinity.

In a further particularly preferred embodiment, the detectable markers for staining the targets are selected, in particular form the pool of detectable markers, such that at least some of the detectable markers have an affinity to more than one of the targets. Specifically, the at least some of the detectable markers each have an affinity to a different one of the more than one of the targets. Thus, the at least some of the detectable markers may each have an affinity to a group of targets. Since the cross-reactivity is considered in form of the affinity information in the unmixing step, this deliberate cross-reactivity of markers enables analysing the biological sample with an increased accuracy.

Preferably, the raw readout data is generated during the step of generating a readout by spectral unmixing with respect to detectable labels of the markers. For example, in case the labels are fluorophores, the fluorescent emission spectra of the fluorophores may overlap. Knowledge of the particular fluorescent emission spectra may be used to unmix spectral data when generating the readout of the biological sample with the markers. This enables analysing the biological sample with an increased accuracy.

Preferably, the affinity information specifying an affinity of each marker towards at least some of the targets is generated experimentally or by modelling prior to the step of providing the affinity information. This enables generating accurate affinity information. In particular, the affinity information may be generated for a pool of detectable markers that are suitable for a particular biological sample based on the expected targets or target molecules in the particular biological sample. The modelling may be performed in-silico and be performed remotely from a person or system carrying out the method. For example, the modelling may be performed on a remote server and generated affinity information may be provided to the person or system carrying out the method.

It is particularly preferred that the affinity information specifying an affinity of each marker towards at least some of the targets is generated by means of a language model. This enables generating accurate affinity information. In particular, the language model may be a large (protein) language model built with, for example, machine learning methods such as artificial neural networks. Training data for the language model may comprise pairs or groups of target or epitopes and affinity reagents or paratopes with a particular degree of affinity to each other. The training data may be generated experimentally of by modelling. A general example of such an approach is the AlphaFold model. Such a language model may further be used to generate markers, in particular the respective affinity reagents or their (amino acid) sequences, that have a desired cross-reactivity characteristic.

Preferably, during the step of unmixing the raw readout data, information about the biological sample is additionally applied to generate the unmixed readout data with respect to the targets. This enables analysing the biological sample with an increased accuracy. For example, if a certain target is known not to be present in a particular biological sample, any cross-reactivity towards that target may be disregarded. The information about the biological sample may be genomic, transcriptomic, proteomic data of the biological sample. This information about the biological sample may be part of the affinity information and applied with the unmixing algorithm.

Preferably, during the step of unmixing the raw readout data, the unmixing algorithm further provides measures of deviation with respect to the targets. This enables providing a measure of confidence associated with the unmixed readout data with respect to the targets. For example, a measure of deviation may be residuals, confidence intervals, or error values. The measure of deviation may further be used to generate a reliability score for a particular experiment. In case the measure of deviation is high, a different set of markers more suitable in terms of their cross-reactivity and affinity information may be suggested to a user.

Preferably, the steps of staining and generating a readout of the biological sample are iteratively repeated, and wherein during each iteration the biological sample is stained with a different set of detectable markers. This enables analysing the biological sample with an increased accuracy. For example, the different set of markers may differ from the markers of a previous iteration only by the association of the labels with the affinity reagents. A particular target may then also be identified by the order of the labels the target was marked with across several iterations. In particular, in this way, the number of labels and affinity reagents may be smaller than the number of targets. Each iteration may further include removing, dissolving and/or disabling the markers of the previous iteration prior to staining with the next set of markers. The markers of the different set of markers may be chosen based on the affinity information of the markers, for example chosen from the pool of detectable markers.

Preferably, the raw readout data is generated during the step of generating a readout based on all readouts of the iteratively generated readouts of the biological sample and unmixing with respect to the markers of the different sets of detectable markers is based on the raw readout data comprising all readouts of the iteratively generated readouts. This enables analysing the biological sample with an increased accuracy.

Preferably, the markers comprise metal labels and the readout generated of the biological sample with the markers is based on the mass of the metal labels. The readout may be generated by means of a mass spectrometer, for example. This enables detecting a wide range of different targets.

Preferably, the markers comprise oligonucleotide labels, in particular barcoded oligonucleotide labels, and the readout generated of the biological sample with the markers is based on a sequence of the oligonucleotide. The readout may be generated by sequencing the oligonucleotide label, for example. This enables detecting a wide range of different targets.

Preferably, the markers comprise an optically detectable label, such as a fluorophore, and the readout generated of the biological sample with the markers is based on light emitted by the optically detectable label. The readout may be generated by means of a cytometer, plate reader, or a microscope, for example. In particular, the readout may be at least one of a fluorescence intensity, a fluorescence lifetime or a photon count. This enables detecting a wide range of different targets.

In a further aspect, a kit of detectable markers being adapted to carry out the method for analysing a biological sample is provided, wherein the kit of detectable markers comprises a plurality of detectable markers, which in particular may be selected from the pool of detectable markers based on their affinity information.

In a further aspect, a system for analysing a biological sample comprising multiple targets is provided. The system comprises means configured to carry out the method according to one of the preceding claims. In particular, the system may comprise at least a controller at least configured to carry out the step of unmixing the raw readout data. The controller may comprise an interface for inputting (additional) affinity information by a user and/or for receiving (additional) affinity information from a database or a cloud service. The controller may further be configured to direct a stainer to carry out the step of staining the targets of the biological sample and to direct a readout device to generate the - preferably iterative - readout of the biological sample with the markers.

The system has the same advantages as the method described above. In particular, the system can be combined with the features of the dependent claims directed to the method.

Short Description of the Figures

Hereinafter, specific embodiments are described referring to the drawings, wherein:

Figure 1     is a flow chart of a method for analysing a biological sample comprising multiple targets,

Figure 2     schematically shows an unmixing algorithm as a set of linear equations for each pixel,

Figure 3     schematically shows affinity information with reference to three affinity reagents,

Figure 4     is a schematic summary of the method,

Figure 5     schematically shows a combination of the method with dye unmixing, and

Figure 6     is a schematic illustration of how cell type or cell state-specific affinity information matrices can be derived by weighting them with cell type or cell state-specific weights.

Detailed Description

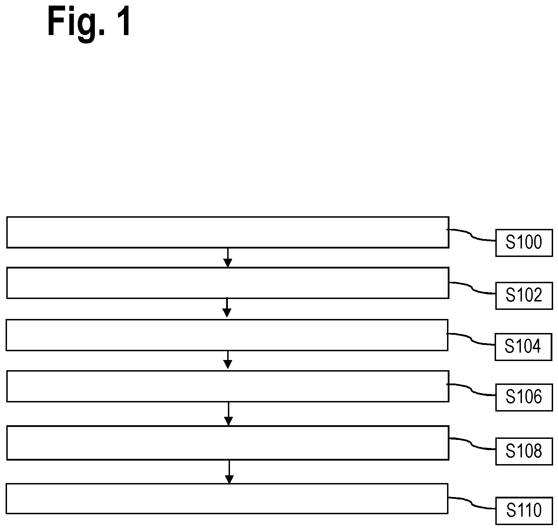

Figure 1 is a flow chart of a method for analysing a biological sample comprising multiple targets. The biological sample may be a tissue section, for example. The targets of the biological sample are parts or an area of the biological sample or its components that markers may bind to. For example, the targets may be epitopes of proteins of the biological samples that markers, in particular antibodies of the markers, may bind to. The targets may therefore be a part of at least one target molecule of the biological sample. A target molecule may comprise a single or several targets.

The method starts in step S100. In step S102 the targets of the biological sample are stained with multiple detectable markers. Staining the targets involves introducing the markers into the biological sample, such that the markers may bind to their respective targets. Each marker comprises an affinity reagent and a detectable label. The affinity reagent is configured to bind to the target and the label is detectable such that the entire marker may be detected. Several copies of each marker may be introduced that bind to a plurality of the target, in particular copies of the target, being present in the biological sample.

An example of an affinity reagent is an antibody or an aptamer. The label may be a fluorophore, for example. Whilst affinity reagents such as antibodies are generally selected to bind specifically and predominately to one target, they frequently exhibit affinities to several further off-targets and may consequently bind to these several further off-targets, often with reduced affinities. Thus, when staining the targets of the biological sample with the detectable markers, each detectable marker may bind to a target that the respective marker is specific to, however, the markers may bind to other targets, specifically the off-targets.

In step S104 affinity information is provided for each marker used to stain the biological sample. The affinity information includes a measure of affinity for each marker towards at least some of the targets. Such a measure of affinity may, for example, be a dissociation constant ($K_D$) for a particular pair of marker and target. The affinity information for each marker may preferably be provided for all of the targets, in particular, this may include off-targets of each marker.

The affinity information may be generated by a large protein language model.

The affinity information may include experimental data, for example, from biochemical assays such as immunoprecipitation coupled with mass spectrometry or phage display. Further, the affinity information may include data from predictions through the analysis of sequence homologies or structural similarities, molecular docking or machine learning, deep learning, and AI-based protein modelling and docking, the affinity information may further derive from literature mining using natural language processing. Similarly, protein structure prediction using approaches such as AlphaFold and molecular docking (e.g. LZerD, ZDOCK, HADDOCK, ClusPro, RosettaDock, HEX, Swarm Dock, ATTRACT, SymmDock, PSI-BLAST or FASTA) may be used to generate affinity information in silico.

For example, the affinity information may be derived from a large protein language model trained using epitope and paratope information, which may be experimentally derived or predicted and then used to predict epitope:paratope interactions to generate affinity information and/or to generate sequences that can be used for affinity reagent de novo generation. In this way affinity reagents may be generated de novo that have desired affinity characteristics, which may then be validated by one of the aforementioned methods (e.g. protein microarrays, phage display). This may in particular be used to predict aptamer sequences for use as affinity reagents. Further this aspect of the method may be used to predict aptamer modifications that allow the formation of a paratope with desired affinity characteristics. The prediction methods above may be used to generate training data that can be used to train a corresponding model.

In step S106 a readout of the stained biological sample with the markers is generated in order to generate raw readout data. The specific type of readout depends on the type of labels the markers comprise. For example, the labels of the markers may be optically detectable and comprise optically detectable labels such as fluorescent dyes. In this case an optical readout may be generated by means of a microscope or a flow cytometer. In particular, the fluorescent dyes may be excited with excitation light and the fluorescent emission light emitted by the fluorescent dyes is captured and detected by a microscope with a suitable detector. All markers specific, or predominantly specific, to a particular one of the targets generally comprise labels with the same optical properties, for example, they have the same excitation and emission wavelength. Markers specific, or predominantly specific, to different targets generally comprise labels with different optical properties in order to be able to optically distinguish between the markers and therefore the targets. The optical readout generated may comprise fluorescent brightness or intensity values and/or fluorescence lifetime of the detected markers, in particular their labels, on a per-pixel basis, for example.

Alternatively, the labels may comprise metals and the readout may be generated by mass spectrometer. The readout may in this case comprise the mass of the label. In a further alternative, the labels may comprise oligonucleotides and the readout may be generated by sequencing the oligonucleotides. The oligonucleotide sequence of a particular marker may be specific to the target the marker specifically or predominantly binds to. Thus, the markers may be distinguished by their sequence.

Thus, the raw readout data may comprise relative or absolute values of the abundance or presence of the markers in the biological sample. For example, the fluorescent intensity values of the labels may be taken as measures for the relative or absolute values of the abundance or presence of the markers in the biological sample.

Additionally, the raw readout data generated in step S106 may optionally be generated by unmixing with respect to the detectable labels of the markers. For example, in the case of fluorescent labels, the raw readout data may be generated by spectral unmixing with respect to the optically detectable labels of the markers.

In step S108 the raw readout data is unmixed with respect to the targets by applying an unmixing algorithm and the affinity information in order to generate unmixed readout data with respect to the targets. Thus, the tendency of a particular marker to only bind a respective target or to bind the respective target predominantly and to bind to other targets or off-targets is taken into account.

For example, the raw readout data associated with a particular pixel or area of the readout may be represented as the signal $S$ of the markers ($m$) with which the biological sample was stained. Each marker comprises an affinity reagent, which has a known affinity or cross-reactivity to the targets in the biological sample. Each target being a part of a different protein of the biological sample, for example. This affinity information of the markers $m$, in particular of the associated affinity reagents, to the targets ($t$) may be represented by the matrix $A$. The unmixed readout data may be represented by the abundance $X$ of each particular target $t$ in the pixel or area of the readout. In short, the signal $S$ in the pixel or area of the readout is determined by the abundance $X$ of the targets $t$ in the pixel or area of the readout and the affinity $A$ of the markers $m$ to the targets $t$:

$$\begin{pmatrix} s_1 \\ \vdots \\ s_m \end{pmatrix} = \begin{pmatrix} a_{11} & \cdots & a_{1t} \\ \vdots & \ddots & \vdots \\ a_{m1} & \cdots & a_{mt} \end{pmatrix} \begin{pmatrix} x_1 \\ \vdots \\ x_t \end{pmatrix}$$

By applying the unmixing algorithm, the abundance $X$ of the targets may be determined from the signal $S$ and the affinity information $A$:

$$\begin{pmatrix} a_{11} & \cdots & a_{1t} \\ \vdots & \ddots & \vdots \\ a_{m1} & \cdots & a_{mt} \end{pmatrix}^{-1} \begin{pmatrix} s_1 \\ \vdots \\ s_m \end{pmatrix} = \begin{pmatrix} x_1 \\ \vdots \\ x_t \end{pmatrix}$$

The method ends in step S110.

As an alternative to the method described above, the detectable markers used for staining in step S102 may be selected from a pool of detectable markers prior to step S102. This selection may comprise considering affinity information of the makers of the pool of detectable markers and the targets of the biological sample. For example, the markers used for staining may be chosen such that they have affinity to only one target and no affinity to the remaining targets of the multiple targets.

Alternatively or additionally, the markers being used for staining may be chosen based on affinity information comprising experimental conditions which might include particulars of a staining protocol, e.g. which buffer is being used or the temperature for the staining process. Preferably, further experimental conditions are advised or predicted based on the affinity information which improve results for the generation of unmixed readout data. Alternatively, the markers may be chosen such that their affinity for the targets other than the predominant target, or the off-targets, is below a predetermined threshold. Thus, cross-reactivity of markers may be minimised.

Alternatively, the markers may be chosen such that the smallest set of markers can be used in the method that allows yielding a well-posed set of linear equations. Thus, the information content of an assay per marker used is maximized and cost per assay per marker is minimized and time to acquire readout data per marker is minimized.

In a further alternative, the steps S102 and S106 may be repeated iteratively. For example, a biological sample may be stained (S102) with different sets of markers and after each staining step, a readout may be generated of the biological sample (S106) with the respective set of markers and raw readout data may be generated. In the step S104 affinity information of all markers of the sets of markers is provided. The step S108 then uses the raw readout data of all iteratively generated readouts of the biological sample in order to generate the unmixed readout data with respect to the targets. For each staining step which is carried out iteratively, the respective markers for particular stainings might be selected.

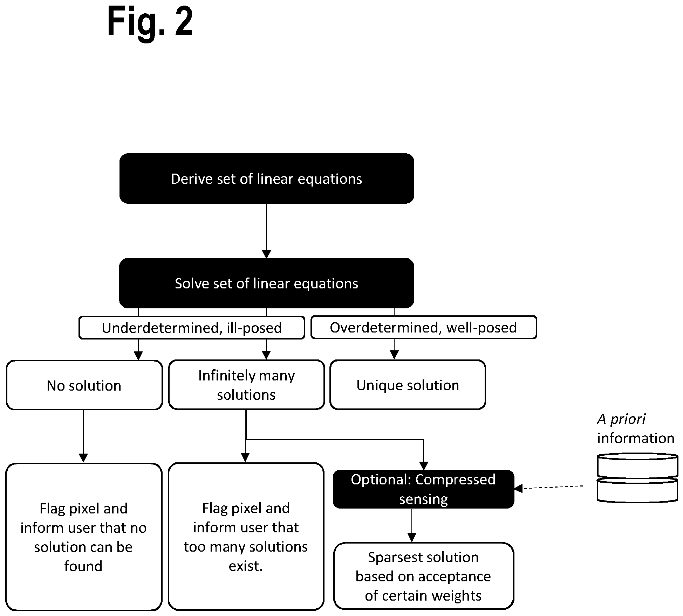

Figure 2 schematically shows an unmixing algorithm as a set of linear equations for each pixel, as an example of an unmixing algorithm. This set may be fully determined or well-posed in which case there will be a unique solution that can be found by using the Gauss elimination algorithm. This set may alternatively have no solution or it may be under determined or ill-posed and have infinitely many solutions. The method may be used to inform the user, when a solution cannot be found, which is a value in itself, as it indicates that a meaningful measurement cannot be performed. Likewise, in case the set of linear equations is ill-posed, the method may be used to inform the user, which may then decide to resort to compressed sensing as an approach and/or may or may not decide to include a priori information into the measurement. A priori information may include known data on target expression, biological sample cell types, cell state, pathway, protein-protein interaction. Like for example, the range of possible solutions may be restricted by gene expression information about the cell type under study, which may also be determined from information of the experiment.

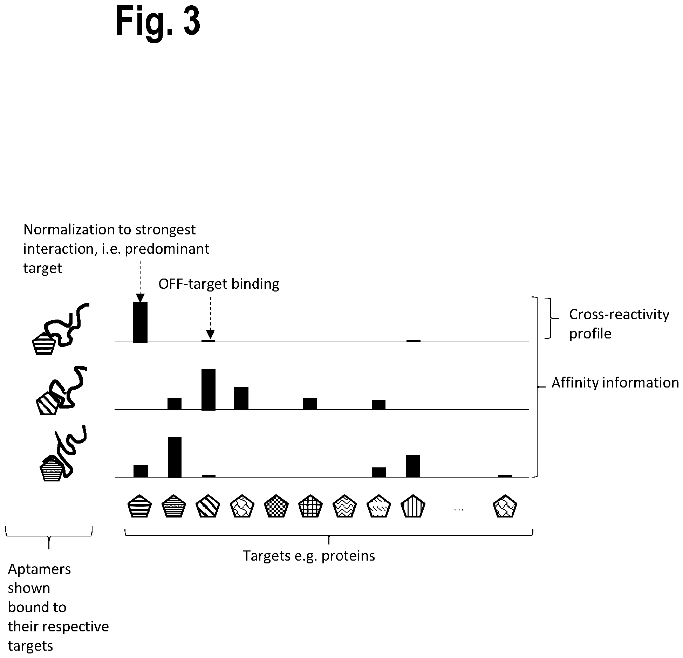

Figure 3 schematically shows affinity information with reference to three affinity reagents, particularly aptamers, bound to their predominant targets, wherein binding to that target is normalized to 1. In this example the difference between the affinities of the said three aptamers is ignored for the sake of simplicity and they are considered to have substantially the same affinity to their respective targets. Alternatively, it is possible to determine the actual affinities, i.e. $K_D$ values and work with them. Further the cross-reactivity profiles contain information about the binding of said aptamers to their OFF-targets. While in this example aptamers are used, this works in an analogous fashion for other affinity reagents like antibodies or nanobodies, for example.

By cross-referencing different experiments with overlapping sets of affinity reagents the method can learn, which cross-reactivity profiles generate the least residuals retrospectively and optimize cross-reactivity profiles and the target unmixing algorithm accordingly. Likewise, as more and more epitope-paratope surface structures become available it may be well possible to (A) predict high quality cross-reactivity information and (B) design specific paratopes that exhibit a desired cross-reactivity profile. This may in particular be the case for aptamers, which are usually nucleic acid based and can be easily synthesized. Taken together a number of methods exist to generate a priori information about the cross-reactivity of affinity reagents. Such affinity information may then be stored on a cloud server, which may also be used to perform target unmixing of user uploaded data.

Further the interaction between targets and affinity reagents is known to be dependent on all conditions that affect epitope-paratope interface ("relevant conditions"), which include but are not limited to ionic strength (e.g. salts), detergents (e.g. Tween-20, Triton X-100), pH (impacts the charge of the epitope or paratope), reducing agents (e.g dithiothreitol. beta-mercaptoethanol), temperature, certain alcohols, chaotropic agents (e.g. urea, guanidinehydrochloride) as well as possible fixation conditions (e.g. paraformaldehyde fixation with varying concentrations, durations vs. other forms of fixation like glutaraldehyde, methanol fixation, cryo-fixation, or non-fixed native samples). Affinity information may be generated specific to for example denatured epitopes or paraformaldehyde fixed epitopes, which may be partially masked, for certain applications like immunofluorescent staining. Affinity information may also be modified based on measurements or prediction. For example, a higher ionic strength may be found to lower the affinity of some affinity reagents to some of the targets. This may be experimentally determined or predicted.

The system may aid in taking this decision by providing estimates of resulting target unmixing quality, i.e. expected residuals and statistical confidence in the unmixing result for a defined set of targets and a given set of affinity reagents and a given assay and set of relevant conditions or in order to define a suitable set of affinity reagents.

The affinity information may also include information about the binding to different isoforms of the same protein. This is particularly important, as up to 95% of human multi-exon genes are alternatively spliced. In fact, the rise in metazoan complexity, while accompanied by a general increase in genome size, is mainly not attributable to an increase in the number of genes, but mostly increasing complexity of regulation. Alternative splicing is one such mechanism and it has been estimated that about 15% of human hereditary diseases and cancers are associated with alternative splicing. The number of human genes is estimated to be around 20,000, while the number of transcript isoforms is around 150,000 (Jiang and Chen 2021; Alternative splicing: Human disease and quantitative analysis from high-throughput sequencing, Computational and Structural Biotechnology Journal, Volume 19, 2021, Pages 183-195, ISSN 2001-0370, https://doi.org/10.1016/j.csbj.2020.12.009.). The reliable identification of protein isoforms is therefore of great importance. Likewise, post-translational modifications (PTMs), such as for example phosphorylation, ubiquitination, sumoylation, glycosylation and other PTMs are key to signal transduction, protein complex formation as well as to the regulation of protein degradation. Cell atlas initiatives have generated large datasets including for example single cell RNA seq data that can be leveraged to derive cell type/cell state-specific consensus expression profiles, that include protein isoforms.

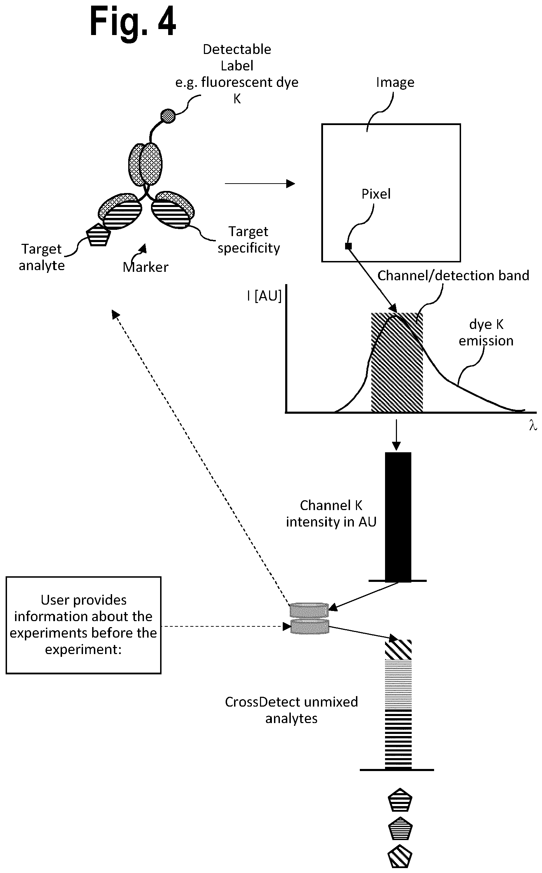

Figure 4 is a schematic summary of the method: A user may optionally provide information about the experiment for example the research question, the targets of interest, sample type, sample origin, developmental stage, physiological condition, electronic health record data, drug treatments, affinity reagents planned to be used in the assay, assay type. The method may then optionally analyse said information and generate a prediction that may be tuned to best quality of the target unmixing, fastest time-to-result, or for example lowest cost of the assay. The method may at this point predict already whether an assay has a high, medium, low or now chance of producing a target unmixing result that exceed user defined quality metrics (e.g. maximum error, level of statistical confidence). The user may then perform an experiment and analyse it or may reanalyse historical data preferably on a cloud server that hosts both the cross reactivity information, experimental data, and target unmixing software. Target unmixing may then lead to a decomposed result.

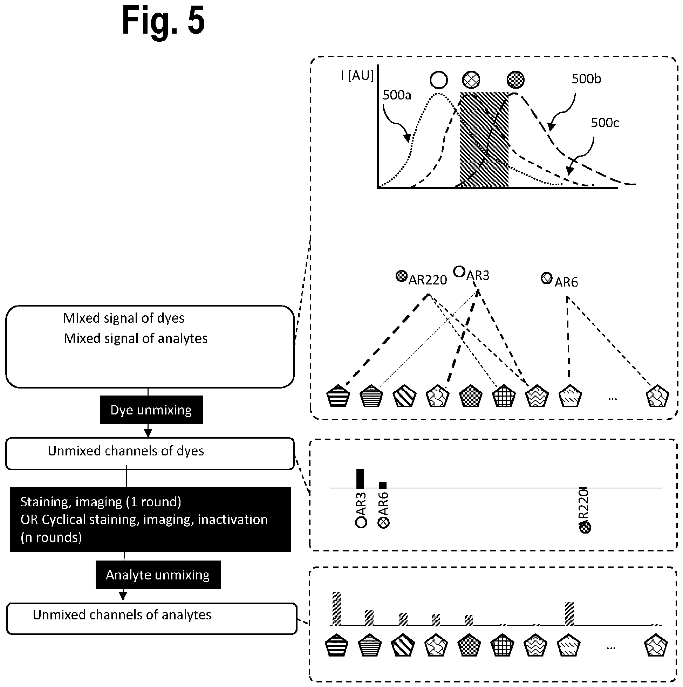

Figure 5 schematically shows a combination of the method with dye unmixing. In this example three dyes are used, whose emission spectra 500a, 500b, 500c overlap to label antibodies AR220, AR3, and AR6 respectively each of which with its own target (thick dashed line) and OFF-target(s) (thiner dashed lines). In a first step dye unmixing is performed leading to "clean channels of dyes" and "residuals". Then target unmixing is performed leading to "clean values of analytes", which includes both target and OFF-target values. A cyclical staining, imaging, and inactivation process may be used to increase plexity of the assay, i.e. each round another set of affinity reagents may be used with the same set of labels.

Further strategies to increasing plexity are also compatible with the method for example the use of combinatorial labels, with e.g. multiple colors, or a hybrid of fluorescent dye and Raman label. Such combinatorial labels may be used in conjunction with a single round readout or with a cyclical staining, imaging, and inactivation process. In particular, they may be used in conjunction the methods and devices described in the following patent applications: WO 2022/242887 A1, the European patent applications with the application numbers EP23178065, EP23155584, EP23183879 and EP23168397, the complete content thereof is incorporated herein by reference.

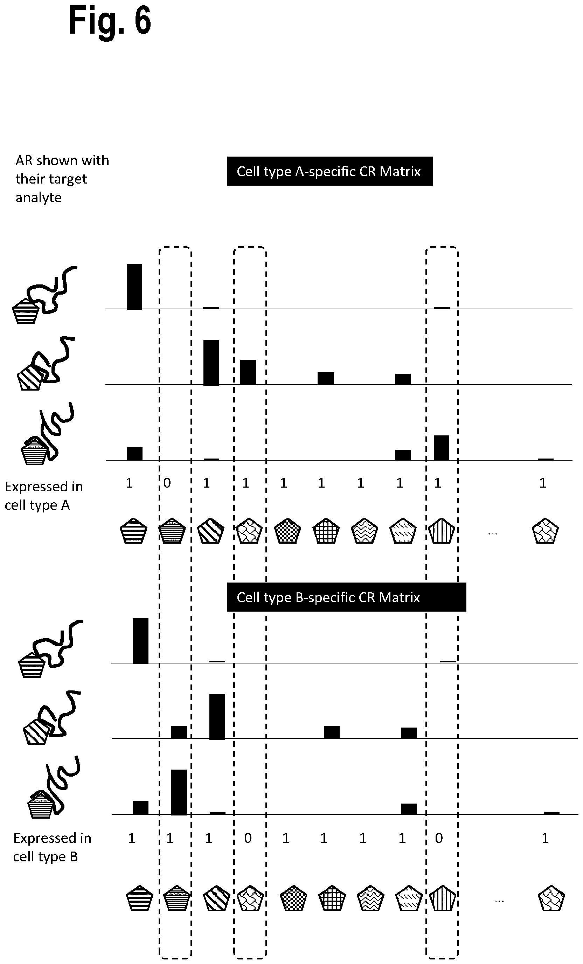

Figure 6 is a schematic illustration of how cell type or cell state-specific affinity information matrices can be derived by weighting them with cell type or cell state-specific weights. Cell atlas initiatives and user-generated single cell RNA sequence data for example, may be used to derive cell type-specific gene expression signatures. These weights may in the easiest case be "1"s and "0"s for genes that are expressed in a given cell-type or genes that are known not be expressed in a certain cell-type. The same applies to splice-isoforms, i.e. protein N isoform K may be expressed in cell type A but isoform L of protein N may not be expressed. Using such profiles cell type-specific affinity information matrices can be generated and used to decompose signals using cell type-specific affinity information. To this end an image analysis step has to be included in the workflow comprising at least one optical readout, which segments individual cells and assigns a certain cell type/state to them. This may greatly facilitate solving the system of linear equations.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Reference Signs

500a, 500b, 500c    Emission spectra

**Claims**

1. A method for analysing a biological sample comprising multiple targets, comprising the following steps:

   staining the targets of the biological sample with multiple detectable markers,
   providing affinity information specifying an affinity of each marker towards at least some of the targets,
   generating a readout of the biological sample with the markers to generate raw readout data, and
   unmixing the raw readout data by applying an unmixing algorithm and the affinity information to generate unmixed readout data with respect to the targets.

2. The method according to claim 1, wherein the targets are on at least one target molecule.

3. The method according to one of the preceding claims, wherein the detectable markers for staining the targets are selected from a pool of detectable markers based on affinity information specifying the affinity of each of the detectable markers in the pool of detectable markers towards the targets of the biological sample.

4. The method according to one of the preceding claims, wherein the detectable markers for staining the targets are selected such that the affinity of each detectable marker to more than one of the targets is below a predetermined threshold.

5. The method according to any one of the one of the preceding claims, wherein the detectable markers for staining the targets are selected such that at least some of the detectable markers have an affinity to more than one of the targets.

6. The method according to one of the preceding claims, wherein the raw readout data is generated during the step of generating a readout by spectral unmixing with respect to detectable labels of the markers.

7. The method according to one of the preceding claims, wherein the affinity information specifying an affinity of each marker towards at least some of the targets is generated experimentally or by modelling prior to the step of providing the affinity information.

8. The method according to claim 7, wherein the affinity information specifying an affinity of each marker towards at least some of the targets is generated by means of a language model.

9. The method according to one of the preceding claims, wherein during the step of unmixing the raw readout data, information about the biological sample is additionally applied to generate the unmixed readout data with respect to the targets.

10. The method according to one of the preceding claims, wherein during the step of unmixing the raw readout data, the unmixing algorithm further provides measures of deviation with respect to the targets.

11. The method according to one of the preceding claims, wherein the steps of staining and generating a readout of the biological sample are iteratively repeated, and wherein during each iteration the biological sample is stained with a different set of detectable markers.

12. The method according to claim 11, wherein the raw readout data is generated during the step of generating a readout based on all readouts of the iteratively generated readouts of the biological sample and unmixing with respect to the markers of the different sets of detectable markers is based on the raw readout data.

13. The method according to one of the preceding claims, wherein the markers comprise metal labels and the readout generated of the biological sample with the markers is based on the mass of the metal labels.

14. The method according to one of the preceding claims, wherein the markers comprise oligonucleotide labels and the readout generated of the biological sample with the markers is based on a sequence of the oligonucleotide.

15. The method according to one of the preceding claims, wherein the markers comprise an optically detectable label and the readout generated of the biological sample with the markers is based on light emitted by the optically detectable label.

16. A kit of detectable markers being adapted to carry out the method of one of the preceding claims, wherein the kit of detectable markers comprises a plurality of detectable markers having the characteristics of one of the claims 3 to 5.

17. A system for analysing a biological sample comprising multiple targets, the system comprising means configured to carry out the method according to one of the preceding claims.

# Fig. 1

```
┌─────────────────────────────────────────────────┐      ┌──────┐
│                                                 │──────│ S100 │
└─────────────────────────────────────────────────┘      └──────┘
                         │
                         ▼
┌─────────────────────────────────────────────────┐      ┌──────┐
│                                                 │──────│ S102 │
└─────────────────────────────────────────────────┘      └──────┘
                         │
                         ▼
┌─────────────────────────────────────────────────┐      ┌──────┐
│                                                 │──────│ S104 │
└─────────────────────────────────────────────────┘      └──────┘
                         │
                         ▼
┌─────────────────────────────────────────────────┐      ┌──────┐
│                                                 │──────│ S106 │
└─────────────────────────────────────────────────┘      └──────┘
                         │
                         ▼
┌─────────────────────────────────────────────────┐      ┌──────┐
│                                                 │──────│ S108 │
└─────────────────────────────────────────────────┘      └──────┘
                         │
                         ▼
┌─────────────────────────────────────────────────┐      ┌──────┐
│                                                 │──────│ S110 │
└─────────────────────────────────────────────────┘      └──────┘
```

# Fig. 2

```
┌─────────────────────────────────────┐
│   Derive set of linear equations     │
└─────────────────────────────────────┘
                  │
                  ▼
┌─────────────────────────────────────┐
│    Solve set of linear equations     │
└─────────────────────────────────────┘
   ┌──────────────────────────┐  ┌──────────────────────────┐
   │  Underdetermined, ill-posed │  │ Overdetermined, well-posed │
   └──────────────────────────┘  └──────────────────────────┘
```

| No solution | Infinitely many solutions | Unique solution |

A priori
information

Flag pixel and inform user that no solution can be found

Flag pixel and inform user that too many solutions exist.

Optional: Compressed sensing

Sparsest solution based on acceptance of certain weights

# Fig. 3

Normalization to strongest interaction, i.e. predominant target

OFF-target binding

Cross-reactivity profile

Affinity information

Targets e.g. proteins

Aptamers shown bound to their respective targets

# Fig. 4

Detectable
Label
e.g. fluorescent dye
K

Image

Target
specificity

Target
analyte

Marker

Pixel

Channel/detection band

I [AU]

dye K
emission

λ

Channel K
intensity in AU

User provides
information about the
experiments before the
experiment:

CrossDetect unmixed
analytes

# Fig. 5

Mixed signal of dyes
Mixed signal of analytes

Dye unmixing

Unmixed channels of dyes

Staining, imaging (1 round)
OR Cyclical staining, imaging, inactivation
(n rounds)

Analyte unmixing

Unmixed channels of analytes

# Fig. 6

AR shown with
their target
analyte

Cell type A-specific CR Matrix

Expressed in
cell type A

| 1 | 0 | 1 | 1 | 1 | 1 | 1 | 1 | 1 | | 1 |

Cell type B-specific CR Matrix

Expressed in
cell type B

| 1 | 1 | 1 | 0 | 1 | 1 | 1 | 1 | 0 | | 1 |

Europäisches Patentamt

European Patent Office

Office européen des brevets

# EUROPEAN SEARCH REPORT

**Application Number**

EP 23 19 0568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/102140 A1 (UNIV CALIFORNIA [US]) 27 May 2021 (2021-05-27) * whole document, in particular p. 4, l. 12-14; p. 15, l. 21-29; p. 27, l. 30-32; p. 35, l. 13 - p. 36, l. 25; p. 42, l. 4-32; p. 76, l. 8 - p. 77, l. 11; p. 77, l. 16-31; fig. 13 * | 1-12, 14-17 | INV. G01N33/557 |
| A | US 2020/283843 A1 (REGEV AVIV [US] ET AL) 10 September 2020 (2020-09-10) * the whole document * | 1-17 | |
| A | US 2021/208076 A1 (CHANG JAE-BYUM [KR] ET AL) 8 July 2021 (2021-07-08) * the whole document * | 1-17 | |
| A | WO 2015/124772 A1 (VENTANA MED SYST INC [US]; HOFFMANN LA ROCHE [CH]) 27 August 2015 (2015-08-27) * the whole document * | 1-17 | |
| A | EP 4 109 333 A1 (LEICA MICROSYSTEMS [DE]) 28 December 2022 (2022-12-28) * the whole document * | 1-17 | TECHNICAL FIELDS SEARCHED (IPC) G01N |
| A | WO 2023/010046 A1 (UNIV CALIFORNIA [US]) 2 February 2023 (2023-02-02) * the whole document * | 1-17 | |

The present search report has been drawn up for all claims

| Place of search | Date of completion of the search | Examiner |
|---|---|---|
| The Hague | 26 October 2023 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 0568

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2023

| Patent document cited in search report | | Publication date | Patent family member(s) | | Publication date |
|---|---|---|---|---|---|
| WO 2021102140 | A1 | 27-05-2021 | CN | 115087747 A | 20-09-2022 |
| | | | US | 2023034263 A1 | 02-02-2023 |
| | | | WO | 2021102140 A1 | 27-05-2021 |
| US 2020283843 | A1 | 10-09-2020 | NONE | | |
| US 2021208076 | A1 | 08-07-2021 | NONE | | |
| WO 2015124772 | A1 | 27-08-2015 | EP | 3108448 A1 | 28-12-2016 |
| | | | EP | 3550514 A1 | 09-10-2019 |
| | | | US | 2016358335 A1 | 08-12-2016 |
| | | | US | 2020034966 A1 | 30-01-2020 |
| | | | US | 2021366109 A1 | 25-11-2021 |
| | | | WO | 2015124772 A1 | 27-08-2015 |
| EP 4109333 | A1 | 28-12-2022 | NONE | | |
| WO 2023010046 | A1 | 02-02-2023 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022242887 A1
- EP 23178065 A
- EP 23155584 A
- EP 23183879 A
- EP 23168397 A

**Non-patent literature cited in the description**

- **BRADBURY** ; **PLUCKTHIN**. Reproducibility: Standardize antibodies used in research. *Nature*, 2015, vol. 518, 27-29
- **JIANG** ; **CHEN**. Alternative splicing: Human disease and quantitative analysis from high-throughput sequencing. *Computational and Structural Biotechnology Journal*, 2021, vol. 19, ISBN 2001-0370, 183-195, https://doi.org/10.1016/j.csbj.2020.12.009